# FASCICULE DE BREVET EUROPEEN

(11) **EP 2 935 179 B1**
(45) Date de publication et mention de la délivrance du brevet: **30.05.2018**
(21) Numéro de dépôt: 13805610.6
(22) Date de dépôt: 05.12.2013
(51) Int. Cl.: C07C 29/60, C07C 31/20, C07C 51/295, C07C 59/08

(54) **TRANSFORMATION DU GLYCÉROL PAR CATALYSE HÉTÉROGÈNE**
GLYCEROLUMWANDLUNG DURCH HETEROGENE KATALYSE
GLYCEROL CONVERSION BY HETEROGENEOUS CATALYSIS

(30) Priorité: 19.12.2012 FR 1262348
(43) Date de publication de la demande: 28.10.2015
(73) Titulaire: Centre National de la Recherche Scientifique (CNRS), 75794 Paris Cedex 16 (FR); Université Claude Bernard Lyon 1, 69100 Villeurbanne (FR)
(72) Inventeur: PINEL, Catherine, F-69007 Lyon (FR); AUNEAU, Florian, F-74960 Cran Gevrier (FR); VILLANDIER, Nicolas, F-87270 Couzeix (FR); BESSON, Michele, F-01700 Les Echets (FR); DJAKOVITCH, Laurent, F-69100 Villeurbanne (FR)
(74) Mandataire: Lavoix
(86) Numéro de dépôt international: PCT/IB2013/060679
(87) Numéro de publication internationale: WO 2014/097040

(56) Documents cités:
- EP-A2- 0 201 957
- CN-A- 101 225 041
- CN-A- 101 695 657
- US-A1- 2012 253 067
- JEROEN TEN DAM ET AL: "Tuning selectivity of Pt/CaCOin glycerol hydrogenolysis A Design of Experiments approach", CATALYSIS COMMUNICATIONS, ELSEVIER SCIENCE, AMSTERDAM, NL, vol. 13, no. 1, 6 juin 2011 (2011-06-06), pages 1-5, XP028257030, ISSN: 1566-7367, DOI: 10.1016/J.CATCOM.2011.06.007 [extrait le 2011-06-13]
- YIHONG SHEN ET AL: "Efficient synthesis of lactic acid by aerobic oxidation of glycerol on Au-Pt/TiO2 catalysts", CHEMISTRY - A EUROPEAN JOURNAL, WILEY - V C H VERLAG GMBH & CO. KGAA, WEINHEIM, DE, vol. 16, no. 25, janvier 2010 (2010-01), pages 7368-7371, XP009146561, ISSN: 0947-6539, DOI: 10.1002/CHEM.201000740 [extrait le 2010-05-18] cité dans la demande

## Description

La présente invention concerne le domaine technique de la synthèse organique par catalyse hétérogène et plus particulièrement la transformation du glycérol en acide lactique.

Une telle catalyse est généralement connue sur des supports solides tel que le charbon et en utilisant des métaux ou des alliages tels que le rhodium, l'iridium, le platine ou un alliage or/platine. Par exemple la synthèse de l'acide lactique à partir du glycérol a été décrite dans Y. Shen et al. Chem. Eur. J .I 2010,16,7368. Cette synthèse réalisée à 90°C, sous 1 bar d'0₂, en présence d'un catalyseur bimétallique Au-Pt/Ti0₂ et de 4 équivalents molaires de base permet d'obtenir un rendement en acide lactique de 44% ce qui ne permet pas d'envisager une application industrielle. Les catalyseurs au platine sur silice sont réputés inactifs pour l'hydrogénolyse du glycérol (cf. C.Montassier et al, Bull. Soc. Chim. Fr.1989,2,148) tandis que les catalyseurs sur charbon tels que ceux de la demande de brevet US2012/0253067 sont généralement décrits comme ayant une plus faible activité pour cette réaction (cf. Y.Kusunoki et al, Catal. Comm. 2005, 6, 645).

CN101695657A décrit un procédé de préparation d'acide lactique à partir du glycérol via une réaction catalysée par un catalyseur hétérogène Pt-Pd sur un support à base de ZrO2 en milieu basique et sous atmosphère d'oxygène.

EP0201957A2 décrit un procédé de préparation d'acide lactique à partir du glycérol via une réaction catalysée par du Pt/C en milieu basique et sous atmosphère inerte.

L'invention a pour but de remédier à ces inconvénients en fournissant un procédé de fabrication de l'acide lactique à un rendement élevé et à des conditions permettant une application industrielle.

Ainsi un premier mode de réalisation de l'invention est l'utilisation d'un catalyseur à base de Pt/ZrO₂, en présence de glycérol dans un milieu réactionnel basique.

Selon un autre mode de réalisation l'invention porte sur un procédé de préparation d'acide lactique comprenant une étape de transformation du glycérol en présence d'un catalyseur hétérogène à base de platine sur un support à base de zircone, en milieu basique sous atmosphère inerte. Ce procédé est par exemple mis en oeuvre en mettant en présence, sous des conditions réactionnelles adéquates, un substrat à base de glycérol et un catalyseur tel que décrit précédemment.

Les expressions « à base de » expriment que d'autres éléments peuvent être présent dans la mesure où l'élément majoritaire en poids est celui qualifié par cette expression. Par « majoritaire » on entend plus de 50% en poids.

Le métal utilisé comme catalyseur comporte plus de 75%, plus particulièrement plus de 95%, en poids de Pt et le support est constitué de plus de 75%, plus particulièrement de plus de 95% en poids de zircone, ZrO₂. Ainsi le catalyseur peut avantageusement être un catalyseur en platine sur zircone.

Le procédé selon l'invention est réalisé en milieu basique. Ce milieu basique peut être obtenu par l'ajout d'une base, préférablement forte, qui peut-être une base de Bronsted, telle la soude, une base organique, telle le DABCO (1,4-diazabicyclo[2.2.2]octane) ou une base solide tel qu'un oxyde mixte de magnésium lanthane. Selon le procédé de l'invention le milieu basique peut comprendre de 0,5 à 5 équivalent molaire d'une base par rapport au glycérol. La quantité de base peut être équimolaire mais il est avantageux que le milieu basique comprenne un léger excès de base, en moles, par rapport au glycérol. Par exemple, le milieu basique comprend avantageusement de 1 à 1,2 équivalent molaire d'une base par rapport au glycérol. La base peut être ajoutée tout au long de la réaction et/ou simplement être ajoutée à un moment donné en une fois. Il est préféré qu'une quantité de base donnée soit mélangée au substrat comprenant le glycérol avant sa mise en présence du catalyseur. Le pH initial peut être ainsi ajusté, par exemple pour être supérieur à 12 ou 13. Un ajout de base pour maintenir la réaction à un pH donné, par exemple supérieur à 11, peut être avantageux. Selon un aspect de l'invention, le milieu basique est obtenu par l'adjonction de NaOH.

Selon un aspect particulier de l'invention l'étape de transformation est effectuée sous pression, par exemple sous pression d'azote, d'hélium ou d'un autre gaz inerte, en tout ou partie à une pression de 5 à 35 bars.

Un des aspects particulièrement préféré de l'invention est que du glycérol brut peut être utilisé lors de l'étape de catalyse. En utilisant du glycérol brut, le procédé permettant d'éviter ou de diminuer les étapes de raffinages requises pour obtenir un produit pur, en particulier lorsque le glycérol est biosourcé, c'est-à-dire obtenu à partir d'huiles végétales ou de graisses animales et en particulier d'une huile extraite d'oléagineux. Ainsi le glycérol brut est un produit pouvant contenir une certaine proportion, par exemple moins de 20%, de substances autres que du glycérol. De telles substances peuvent être de l'eau, des cendres et/ou des résidus de matières grasses.

Selon un autre aspect préféré de l'invention la quantité de glycérol utilisée lors de l'étape de transformation est au moins 25% (en moles) des produits de départ, préférablement d'au moins 50%.

Il est également préféré que la réaction s'effectue en présence d'un solvant protique telle que l'eau.

La réaction peut avantageusement s'effectuer, en tout ou partie, à une température choisie de 160°C à 250°C, préférentiellement de 165 à 185°C comme par exemple 170°C± 3°C et/ou 180°C ± 3°C. De manière préférentielle la réaction s'effectue en tout ou partie à une température inférieure à 180°C.

Selon un autre aspect de l'invention, le procédé peut en outre permettre d'obtenir du 1,2-propanediol, 1,3-propanediol et/ou de l'acide formique.

Avantageusement, le procédé selon l'invention présente un rendement en acide lactique supérieurs à 55%, de préférence supérieur ou égal à 60% et notamment supérieur à 65%, voire 70 %.

Selon un autre mode de réalisation le procédé selon l'invention met en oeuvre un catalyseur à base de platine sur un support à base d'alumine dans des conditions réactionnelles similaires ou semblables à celles décrites précédemment.

### DESCRIPTION DES FIGURES

L'invention sera mieux comprise à la lecture des figures annexées, qui sont fournies à titre d'exemples et ne présentent aucun caractère limitatif, dans lesquelles :

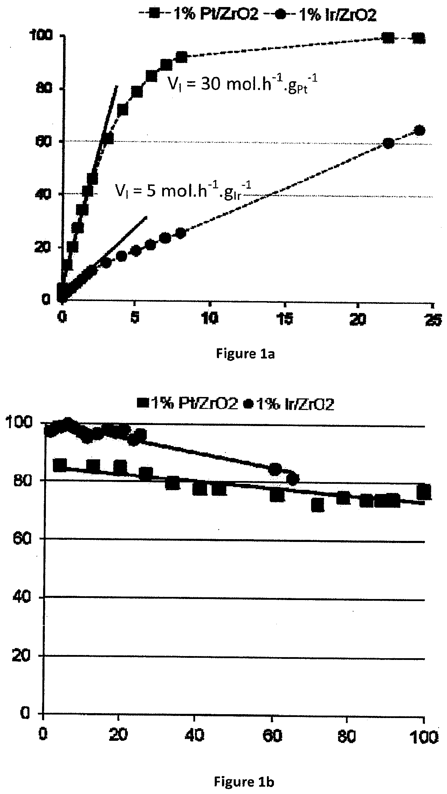

La Figure 1a représente l'évolution du taux de conversion du glycérol (%) en fonction du temps (h) et la Figure 1b représente la sélectivité en acide lactique (%) en fonction de la conversion du glycérol (%) pour les catalyseurs 1% Ir/ZrO2 (rond noirs) et 1% Pt/ZrO2 (carré noir) sous atmosphère d'hélium selon le procédé décrit à l'exemple 1.
Les Figures 2 et 3 représentent respectivement l'évolution de la conversion du glycérol et des rendements en acide lactique, 1,2-propanediol, 1,3-propanediol et acide formique formés au cours de la réaction réalisée en présence d'un catalyseur Platine/Zircone (Figure 2) et en présence d'un catalyseur Platine/Charbon (Figure 3).
Les Figures 4 et 5 représentent l'évolution de la conversion du glycérol et des rendements en produits de la réaction de l'exemple 1 à 5% molaire de glycérol (Figure 4) et à 25% molaire de glycérol (Figure 5).
La Figure 6 représente l'évolution de la conversion du glycérol et des rendements en produits de la réaction réalisée en présence d'un catalyseur Platine/Zircone à partir du glycérol brut.

### EXEMPLES

### Exemple 1 : Synthèse de l'acide lactique selon le procédé de l'invention et étude comparative avec un autre catalyseur sur zircone.

Des catalyseurs Ir/ZrO2 et Pt/ZrO2 (1% massique en métal) ont été préparés par imprégnation d'un support ZrO2 qui est une zircone monoclinique qui possède une surface BET de 52,9 m2/g, un volume poreux de 0,3 mL/g et un diamètre de pores comprit entre 16 et 60 A (Saint-Gobain, Zircone NorPro 50 m²/g). L'imprégnation a été effectuée par une solution aqueuse à partir respectivement des précurseurs IrCl₃ et H₂PtCl₆, 6H₂O. Après calcination à 600°C sous un flux d'air pendant 12 h et réduction à 300°C sous H₂ pendant 3 h, des catalyseurs ayant une charge métallique comparable (ca 1% poids) ont été obtenus. Une étude cinétique a été menée pour comparer leur activité dans des conditions de référence (100ml, d'une solution aqueuse de 5% glycérol/NaOH 1M, 180°C, 30 bar gaz inerte, 500 mg 1% M/ZrO2). Les réactifs sont introduits dans un autoclave en inox équipé avec un pot en Téflon® et soumis à une pression initiale en He de 25 bar (Pf = 30 bar). Le mélange est soumis à une agitation constante à une température de 180 °C.

Le glycérol est converti plus rapidement en présence du catalyseur 1% Pt/ZrO2 qu'à l'aide du catalyseur 1% Ir/ZrO2. En effet, l'activité initiale du catalyseur Pt/ZrO2 est 6 fois plus grande que celle du catalyseur Ir/ZrO2. Après 24h de réaction, les conversions atteignent respectivement 100% et 65% (Tableau 2, entrées 1 et 2).

L'acide lactique est le produit majoritaire observé quel que soit le catalyseur utilisé. La formation d'acide lactique conduit à une légère diminution du pH de la solution. De plus, l'analyse COT montre dans les deux cas, que 100% du carbone organique est en solution, ce qui tend à démontrer que les éventuels phénomènes de reformage en phase aqueuse n'ont lieu que de manière marginale. Ce résultat est cohérent avec l'absence de produits carbonés au sein de la phase gazeuse.

L'analyse du milieu réactionnel en fin de réaction par ICP montre qu'il n'y pas de lixiviation ni du Zr ni du Pt en solution au cours de la réaction (CPt et CZr < 0,1 mg.L-1). Cette observation démontre que la production d'acide lactique en présence de Pt/ZrO2 est due à un procédé catalytique hétérogène et qu'il est donc possible d'envisager un recyclage du catalyseur. Sous atmosphère d'hydrogène (Tableau 1, entrée 3), la conversion du glycérol en présence du catalyseur 1% Pt/ZrO2 est la même, mais le rendement en AL diminue de 78% à 67%. Cette diminution est accompagnée dans le même temps d'une augmentation du rendement en 1,2-propanediol qui passe de 7% à 23%.

**Tableau 1 : Comparaison des catalyseurs 1% Ir/ZrO₂ et 1% Pt/ZrO₂**

| No. | Cat. | pHᵢ | pH_{f} | Conv. (%) | Bilan carbone (%)^{(a)} | Rendement (%)^{(b)} | | |
|---|---|---|---|---|---|---|---|---|
| | | | | | | AL^{(c)} | 1,2-PDO^{(c)} | Autres^{(c)} |
| 1 | 1% Pt/ZrO₂ | 12,9 | 12,5 | 100 | 100 | 78 | 7 | AF : 7, 1,3-PDO : 2, EG : 2, AA et EtOH : < 1 |
| 2 | 1% Ir/ZrO₂ | 13,1 | 12,6 | 65 | 100 | 53 | 3 | AF : 2, 1,3-PDO : 1, EG, AA et EtOH : < 1 |
| 3 | 1% Pt/ZrO2^{(d}} | 13,1 | 12,6 | 96 | 100 | 67 | 23 | AF : 3, 1,3-PDO : 3, EG : 2, AA et EtOH : < 1 |

| | | | | | | | | |
|---|---|---|---|---|---|---|---|---|
| 100 mL 5% glycérol/NaOH 1 M, 500 mg catalyseur, 25 bar de pression initiale en He (Pf = 30 bar), 180°C, 24 h. (a) Déterminé par l'analyse du COT. (b) Déterminé par HPLC. (c) AL : acide lactique ; 1,2-PDO : 1,2-propanediol ; 1,3-PDO : 1,3-propanediol ; AA : acide acétique ; AF : acide formique ; EG : éthylène glycol et EtOH : éthanol. (d)Réaction réalisée sous une pression d'H2 de 50 bar. | | | | | | | | |

### Exemple 2 : Etude comparative de réactions de synthèse d'acide lactique à partir de glycérol en présence d'un catalyseur à base de Platine sur différents support : Pt/ZrO2 et Pt/C.

Les rendements et sélectivités de la synthèse d'acide lactique selon l'invention telle que décrite dans l'exemple 1, ont été comparés à ceux d'une réaction utilisant également un catalyseur hétérogène à base de platine mais sur un support différent : le charbon. Le catalyseur Pt/C est connu pour avoir un très bon rendement lors de cette réaction.

Le catalyseur Pt/C utilisé a été synthétisé selon une méthode décrite dans la littérature P. Korovchenko, C. Donze, P. Gallezot, M. Besson, Catal Today, 2007, 121, 13, à partir d'un charbon CECA L3S possédant une surface BET de 900m²/g. Le catalyseur Pt/C ainsi obtenu possède des particules de l'ordre du nanomètre, par exemple de 1 à 3nm, non détectables par analyse DRX. La synthèse d'acide lactique en présence de Pt/C a été réalisée sous des conditions de réactions équivalentes aux conditions de référence de l'exemple 1. Il a été mélangé 100 mL de glycérol à 5%, 1,8 équivalent de NaOH, avec 161 mg de catalyseur Pt/C à 3%. Le mélange a été porté à 180 °C, sous une pression d'hélium de 30 bar durant 25h.

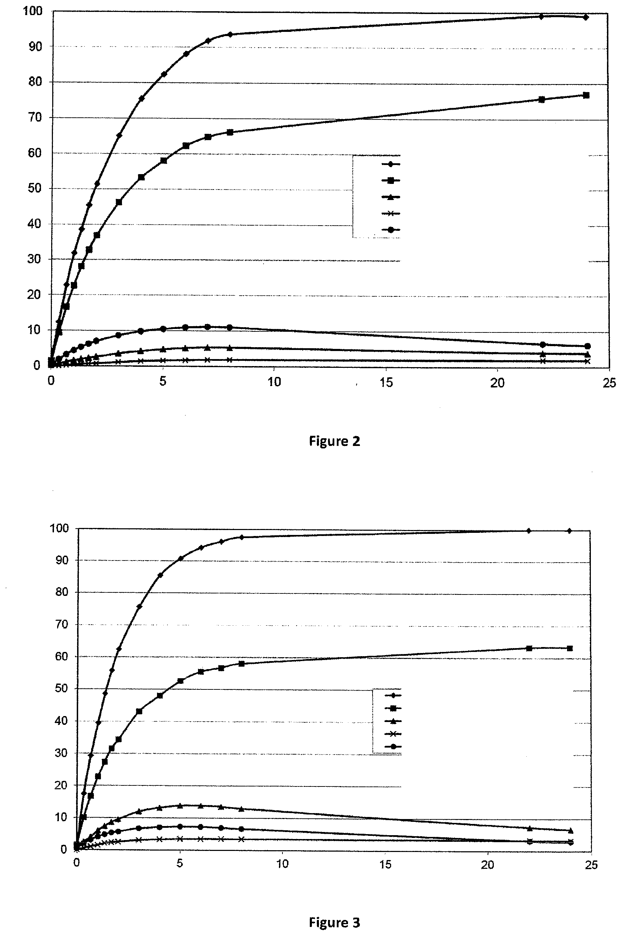

Les résultats de l'étude cinétique de la conversion en glycérol et des rendements en produits formés, effectuée au cours des 25h de réaction, sont présentés dans la figure 2 pour la réaction selon l'invention en présence d'un catalyseur Pt/ZrO₂ et dans la figure 3 pour la réaction avec le catalyseur Pt/C. Les courbes représentent le taux de conversion du glycérol (losange noir), le rendement en acide lactique (carré noir), le rendement en acide formique (rond noir), le rendement en 1,2-propanediol (triangle noir), et le rendement en 1,3-propanediol (croix noire). Dans ces deux figures, le temps, en abscisse, est exprimé en heure et le taux de conversion ou de rendement en produit de la réaction, en ordonnée, est exprimé en %. On peut observer que la courbe (losange noir) de la Figure 2, représentant la conversion du glycérol s'approche des 100% en 25h avec des valeurs avoisinant 98-99% au bout de 23h de réaction. La courbe (carré noir) représentant le rendement en acide lactique croit de façon exponentielle jusqu'à une valeur proche de 78% au bout de 25h de réaction. Les autres produits de la réaction, le 1,2-propanediol, le 1,3-propanediol et l'acide formique (courbes (rond noir), (triangle noir) et (croix noire)) ont des rendements faibles inférieurs à 10%.

On peut observer que la courbe (losange noir) de la Figure 3 représentant la conversion du glycérol atteint au bout de 20h de réaction les 100% de conversion. Toutefois, la courbe (carré noir) représentant le rendement en acide lactique croit de façon exponentielle jusqu'à une valeur proche de 62% à l'issu de la réaction. Les autres produits de la réaction, le 1,2-propanediol, le 1,3-propanediol et l'acide formique (courbes (rond noir), (triangle noir) et (croix noire)) ont des rendements faibles inférieurs à 15% à 5h de réaction.

Cette étude comparative montre que le catalyseur Pt/C est légèrement plus actif en termes de conversion du glycérol que Pt/ZrO2. Toutefois, il apparait que la sélectivité vis-à-vis de l'acide lactique est supérieure de 10% avec un support zircone qu'avec un support charbon, tout au long de l'avancement de la réaction.

### Exemple 3 : Synthèse de l'acide lactique selon le procédé de l'invention et impact de la concentration en glycérol.

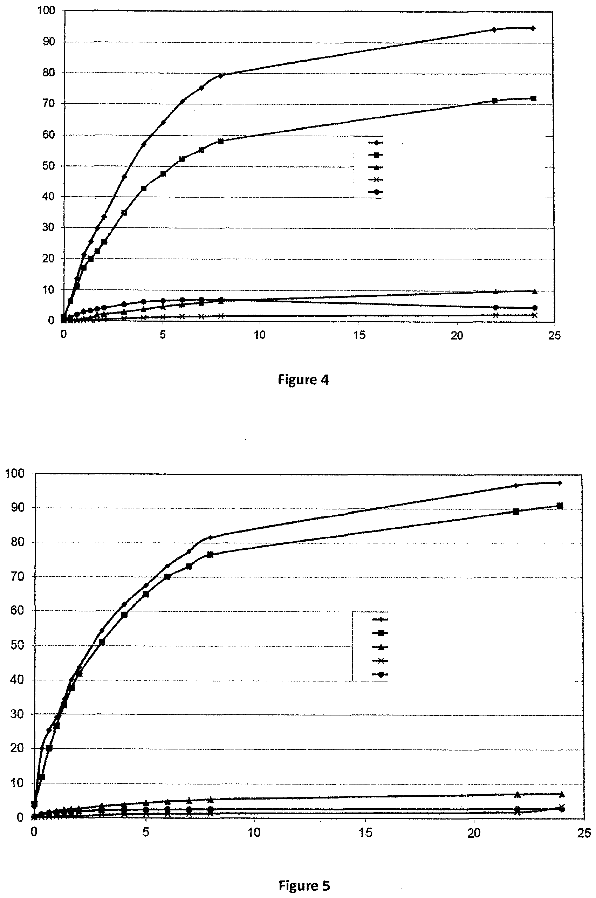

L'impact de la concentration de départ en glycérol sur la réaction selon l'invention telle que décrite dans l'exemple 1, a été étudié. Pour cela, la conversion du glycérol et les rendements en produits formés ont été déterminés par analyse cinétique pour la réaction effectuée dans l'exemple 1, à 5% molaire de glycérol (réaction (a), et pour une réaction identique mais réalisée avec 25% de glycérol (réaction (b). Les deux réactions ont été menées avec le même lot de catalyseur. Pour la première réaction (a), il a été mélangé 100 mL de glycérol à 5% avec 1,1 équivalent de NaOH en présence de 500 mg de Pt/ZrO2 (1 wt.%). La réaction a été menée à 180 °C durant 25h à une pression de 30 bar d'hélium. Pour la deuxième réaction (b), il a été mélangé 100 mL de glycérol à 25% molaire avec 1,1 équivalent de NaOH en présence de 500 mg de Pt/ZrO2 (1 wt.%). La réaction a été menée à 180°C durant 25h à une pression de 30 bar d'hélium. Le rapport NaOH/Gly a été maintenu constant par un ajout de soude. Les légendes des Figures 4 et 5 sont les mêmes que celles utilisées pour les Figures 2 et 3.

On observe que l'évolution de la conversion est semblable pour ces taux de glycérol, et est supérieure à 95%. Toutefois, on remarque de façon surprenante que la sélectivité en acide lactique est nettement améliorée avec une solution à 25% en glycérol. En effet celle-ci atteint 90% au lieu de 80% avec une solution en glycérol de 5%. Par ailleurs, il est à noter que dans ce cas le pH final de la solution est maintenu supérieur à 12, conditions qui favorisent la sélectivité en acide lactique.

### Exemple 4 : Synthèse de l'acide lactique selon le procédé de l'invention pour divers produits de départ.

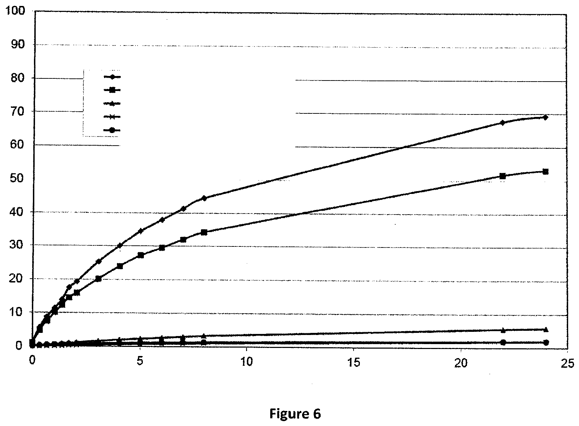

L'impact de la source en glycérol (purifié ou non (brut)) sur la réaction selon l'invention telle que décrite dans l'exemple 1, a été étudié. Pour cela, la conversion du glycérol et les rendements en produits formés obtenus dans l'exemple 1, ont été comparés avec ceux obtenus par analyse cinétique pour une réaction effectuée dans les mêmes conditions de référence mais avec du glycérol brut, non raffiné. Dans ce cas du glycérol brut provenant de la transestérification d'huile de colza a été obtenu. Cette huile contenait, 83,84% en poids de glycérol, 11,86 % en poids d'eau, 3,95% en poids de cendres, 0,33% en poids de matières grasses diverses et 0,017% d'éthanol Le catalyseur utilisé est le Pt/Zr0₂ décrit à l'exemple 1. Pour la réaction à partir de glycérol brut, il a été mélangé 100 mL de glycérol brut à 5% **molaire** avec 1,1 équivalent de NaOH en présence de 500 mg de Pt/ZrO2 (1 wt.%). La réaction a été menée à 180°C, durant 25h à une pression de 30 bar d'hélium. L'activité de Pt/ZrO2 pour la transformation de glycérol brut est plus faible (cf. Figure 6), présentant une diminution de la conversion du glycérol d'environ un facteur 2 (conversion à 5h : 64 et 35%). Toutefois, la sélectivité en acide lactique reste similaire (c'est-à-dire d'environ 75%). Les légendes de la figure 6 sont les mêmes que celles utilisées pour les figures 2 à 5.

### Exemple 5 : Synthèse de l'acide lactique selon le procédé de l'invention à diverses concentrations de NaOH et températures.

Les rendements et sélectivités de la synthèse d'acide lactique selon l'invention telle que décrite dans l'exemple 1, ont été comparés à ceux de réactions utilisant différents rapports NaOH/Glycérol. Il a été mélangé dans autoclave en inox équipé avec un pot en Téflon®, 100 mL d'une solution à 5% de glycérol avec de 0 à 1,8 M de NaOH, en présence de 500 mg de catalyseur 1% Pt/ZrO2. La réaction a été menée sous une pression initiale d'hélium de 15 bar durant 24 h à des températures de 180°C et de 220°C. La conversion du glycérol, les rendements de produits formés ainsi que le pH initial et final, et le bilan carbone de la réaction ont été déterminés pour l'ensembles des réactions ci-dessous. Deux réactions ont été menées à une température de 180°C: la première en présence d'un mélange équimolaire NaOH/glycérol (tableau 2, entrée 1) et la seconde en présence d'un léger excès de base correspondant à 1,1 équivalent de NaOH (tableau 2, entrée 2). La première réaction atteint une sélectivité en acide lactique de 69% pour une conversion de 89% (61% Rdt). La seconde réaction faite en présence d'un léger excès de base, présente une conversion de 95% et une sélectivité en acide lactique (74% Rdt) encore meilleures. Quatre autres réactions ont été menées à une température de 220°C avec des rapports NaOH/glycérol de 0 (tableau 2, entrée 3); 1,2 (tableau 2, entrée 4) ; 1 (tableau 2, entrée 5) et 1,8 (tableau 2, entrée 6). La réaction (2) a une conversion et sélectivité similaire à la réaction (6) tout en utilisant une quantité moindre de NaOH. Le bilan en produits carbonés en solution est complet et aucune trace de CO₂ n'est détectée dans la phase gaz. Il donc possible de réaliser l'hydrogénolyse sélective du glycérol en acide lactique en présence d'un faible ratio NaOH/Gly et du catalyseur 1% Pt/ZrO₂.

La réaction (3) a une bonne conversion (74%) toutefois, la sélectivité pour l'acide lactique est faible, avec une rendement en acide lactique de 9%. Dans ce cas, les produits majoritaires de la réaction sont dans l'ordre le 1,2-PDO (41%) et l'hydroxyacétone (10%). De façon surprenante, lorsque le ratio NaOH/Gly est légèrement augmenté de 0 à 0,2, la conversion diminue légèrement (64%). Cependant dans le même temps, la sélectivité en AL passe de 12% à 38% et l'acide lactique devient le produit majoritaire (4). Les réactions (5) et (6) ont des conversions respectives de 99% et de 100% observées après 24 heures. Ces ratios permettent d'augmenter sensiblement le rendement en acide lactique qui atteint respectivement 65% et 70%. Cependant de manière inattendue des conditions de pH et de températures réduites permettent les meilleurs résultats. On a constaté que l'augmentation de la sélectivité en acide lactique avec le ratio NaOH/Gly s'accompagne d'un accroissement régulier de la proportion d'hydrogène dans la phase gaz.

Quel que soit le ratio NaOH/Gly, le bilan de carbone organique en solution n'est jamais de 100% à 220°C. Ces résultats tendent à démontrer qu'à cette température, les phénomènes de reformage en phase aqueuse ont lieu.

**Tableau 2 : Influence du ratio NaOH/Glycérol en présence du catalyseur 1% Pt/ZrO2**

| n° | NaOH/Gly. (mol/mol) | T (°C) | pHi | pHf | Conv. (%) | Bilan carbone (%)^{a} | Rendement (%)^{b} | | |
|---|---|---|---|---|---|---|---|---|---|
| | | | | | | | AL^{c} | 1,2-PDO^{c} | Autres^{c} |
| 1 | 1 | 180 | 12,9 | 9,5 | 89 | 99 | 61 | 10 | AF(5), EG(2), 1,3-FDO(2), AA (1) |
| 2 | 1,1 | 180 | 13,0 | 11,3 | 95 | 100 | 74 | 10 | AF(6), EG(3), 1,3-FDO(2), AA(1) |
| 3 | 0 | 220 | - | 3,2 | 74 | 89 | 9 | 41 | HA(10), EtOH(3), EG(2), AA(1), 1-PO(1) |
| 4 | 0,2 | 220 | 12,6 | 4,2 | 64 | 86 | 24 | 20 | HA(6), EtOH(3), EG(2), AA(1) |
| 5 | 1 | 220 | 13,1 | 8,9 | 100 | 91 | 65 | 6 | AF(6), EG (2), 1,3-PDO(2), AA(1) |
| 6 | 1,8 | 220 | 13,0 | 12,4 | 100 | 89 | 70 | 2 | AF (5), EtOH(1) 1,3-PDO(2), AA(2), |

| | | | | | | | | | |
|---|---|---|---|---|---|---|---|---|---|
| ₐ Le bilan carbone a été déterminé par l'analyse du COT. _{b} Determiné par HPLC. _{c} AL : acide lactique 1,2-PDO : 1,2-propanediol ;. 1,3-PDO : 1,3-propanediol, AA : acide acétique, AF : acide formique, EG : éthylène glycol, EtOH : éthanol HA : hydroxyacétone. | | | | | | | | | |

## Revendications

1. Procédé de préparation d'acide lactique comprenant une étape de transformation du glycérol en présence d'un catalyseur hétérogène à base de platine sur un support à base de zircone en milieu basique sous atmosphère inerte, ledit catalyseur comportant plus de 75% en poids de Pt et ledit support étant constitué de plus de 75% en poids de zircone, ZrO₂.

2. Procédé selon la revendication 1, **caractérisé en ce que** le catalyseur soit un catalyseur en platine sur zircone.

3. Procédé selon l'une quelconque des revendications 1 à 2, **caractérisé en ce que** le milieu basique comprenne de 0,5 à 5 équivalent molaire d'une base par rapport au glycérol.

4. Procédé selon l'une quelconque des revendications 1 à 3, **caractérisé en ce que** le milieu basique comprenne un léger excès de base, en moles, par rapport au glycérol, ledit milieu basique comprenant de 1 à 1,2 équivalent molaire d'une base par rapport au glycérol.

5. Procédé selon l'une quelconque des revendications 1 à 4, **caractérisé en ce que** le milieu basique est obtenu par l'adjonction de NaOH.

6. Procédé selon la revendication 5, **caractérisé en ce que** ladite étape de transformation soit effectuée sous pression d'azote.

7. Procédé selon l'une quelconque des revendications 1 à 6, **caractérisé en ce que** du glycérol brut est utilisé lors de l'étape de transformation et **en ce que** la quantité de glycérol utilisée lors de l'étape de transformation est au moins 25% (en moles) des produits de départ.

8. Procédé selon l'une quelconque des revendications 1 à 7, **caractérisé en ce que** l'étape de transformation s'effectue en tout ou partie à une température allant de 170°C et 250°C, de préférence inférieure à 180°C.

9. Procédé selon l'une quelconque des revendications 1 à 8, **caractérisé en ce que** l'étape de transformation est effectuée à une pression allant de 5 à 35 bars.

## Patentansprüche

1. Verfahren zum Herstellen von Milchsäure, aufweisend einen Schritt des Umwandelns von Glycerin in Anwesenheit eines auf Platin basierenden heterogenen Katalysators auf einem Zirkonoxid-basierten Träger in einem basischen Milieu unter inerter Atmosphäre, wobei der Katalysator mehr als 75 Gew.-% Pt aufweist und der Träger aus mehr als 75 Gew.-% Zirkonoxid, ZrO₂, gebildet ist.

2. Verfahren gemäß Anspruch 1, **dadurch gekennzeichnet, dass** der Katalysator ein Katalysator aus Platin auf Zirkonoxid ist.

3. Verfahren gemäß irgendeinem der Ansprüche 1 bis 2, **dadurch gekennzeichnet, dass** das basische Milieu 0,5 bis 5 Moläquivalent einer Base im Verhältnis zum Glycerin aufweist.

4. Verfahren gemäß irgendeinem der Ansprüche 1 bis 3, **dadurch gekennzeichnet, dass** das basische Milieu einen leichten Basenüberschuss in Mol im Verhältnis zum Glycerin aufweist, wobei das basische Milieu 1 bis 1,2 Moläquivalent einer Base im Verhältnis zum Glycerin aufweist.

5. Verfahren gemäß irgendeinem der Ansprüche 1 bis 4, **dadurch gekennzeichnet, dass** das basische Milieu durch Hinzufügen von NaOH erhalten wird.

6. Verfahren gemäß Anspruch 5, **dadurch gekennzeichnet, dass** der Schritt des Umwandelns unter Stickstoffdruck durchgeführt wird.

7. Verfahren gemäß irgendeinem der Ansprüche 1 bis 6, **dadurch gekennzeichnet, dass** beim Schritt des Umwandelns Rohglycerin verwendet wird und dass die beim Schritt des Umwandelns verwendete Menge an Glycerin mindestens 25 % (in Mol) der Ausgangsprodukte beträgt.

8. Verfahren gemäß irgendeinem der Ansprüche 1 bis 7, **dadurch gekennzeichnet, dass** der Schritt des Umwandelns vollständig oder teilweise bei einer Temperatur ab 170°C und 250°C, vorzugsweise kleiner als 180°C durchgeführt wird.

9. Verfahren gemäß irgendeinem der Ansprüche 1 bis 8, **dadurch gekennzeichnet, dass** der Schritt des Umwandelns bei einem Druck ab 5 bis 35 Bar durchgeführt wird.

## Claims

1. Process for the preparation of lactic acid, comprising a step of converting glycerol in the presence of a platinum-based heterogeneous catalyst on a zirconia-based support in a basic medium under an inert atmosphere, said catalyst comprising more than 75% by weight Pt and said support being composed of more than 75% by weight zirconia, ZrO₂.

2. Process according to claim 1, **characterised in that** the catalyst is a platinum on zirconia catalyst.

3. Process according to either claim 1 or claim 2, **characterised in that** the basic medium comprises from 0.5 to 5 molar equivalents of a base, relative to the glycerol.

4. Process according to any one of claims 1 to 3, **characterised in that** the basic medium comprises a slight excess of base, in moles, relative to the glycerol, said basic medium comprising from 1 to 1.2 molar equivalents of a base, relative to the glycerol.

5. Process according to any one of claims 1 to 4, **characterised in that** the basic medium is obtained by adding NaOH.

6. Process according to claim 5, **characterised in that** said conversion step is carried out under nitrogen pressure.

7. Process according to any one of claims 1 to 6, **characterised in that** crude glycerol is used in the conversion step, and **in that** the amount of glycerol used in the conversion step is at least 25% (in moles) of the starting products.

8. Process according to any one of claims 1 to 7, **characterised in that** the conversion step is carried out wholly or partially at a temperature ranging from 170°C to 250°C, preferably below 180°C.

9. Process according to any one of claims 1 to 8, **characterised in that** the conversion step is carried out at a pressure ranging from 5 to 35 bar.
